# EUROPEAN PATENT APPLICATION

(11) **EP 1 552 740 A1**
(43) Date of publication of application: **13.07.2005**
(21) Application number: 04290065.4
(22) Date of filing: 09.01.2004
(51) Int. Cl.: A01K 67/027, C12N 9/64, C12N 15/63, A61K 49/00

(54) **Chimeric murine model comprising human hepatocytes - use for screening compounds**

(71) Applicant: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventor: Druilhe, Pierre, 75015 Paris (FR); Hez, Stéphane, 75015 Paris (FR); Kremsdorf, Dina, 75013 Paris (FR); Brechot, Christian, 75015 Paris (FR); Morosan, Serban, 75012 Paris (FR); Lunel, Françoise, 49125 Briollay (FR); Renia, Laurent, 75011 Paris (FR)
(74) Representative: Desaix, Anne

(57) **Abstract**

The invention relates to a method of making a chimeric murine model comprising (a) obtaining an immunocompromised murine host, which carries a transgene or a genetic defect responsible for murine hepatocyte death, (b) implanting human hepatocytes therein, (c) controlling non-adaptive defences and (d) recovering a chimeric murine model harbouring settled human hepatocytes capable of maintaining, differentiating and growing.

The invention also discloses a chimeric murine model and relates to applications in pathogen studies having recourse to said model, including screening compounds or assessing efficacy of compounds in the treatment of pathogen infections or detrimental effects resulting from said infection. The invention also concerns the use of said model to evaluate the interest of compounds in treatment of patients, or in the study of metabolism of compounds contacted with said human hepatocytes.

## Description

### FIELD OF THE INVENTION

The present invention provides a method of making a chimeric murine model comprising human hepatocytes. The invention also discloses a chimeric murine model. The invention also relates to applications in pathogen studies having recourse to said model, including screening compounds or assessing efficacy of compounds in the treatment of pathogen infections or detrimental effects resulting from said infection. The invention also concerns the use of said model to evaluate the interest of compounds in treatment of patients.

The chimeric murine model comprising human hepatocytes can further be useful for the study of metabolism of said human hepatocytes, when said cells are submitted to contact with various agents including drug compounds or drug candidates.

### BACKGROUND OF THE INVENTION

Disease-causing pathogens include microorganisms encompassing viruses, bacteria, fungi or parasites. Other pathogens can be substances inducing or favouring toxic or detrimental reactions to emerge or to spread in hosts, said substances including components derived from microorganisms or produced by the same or can be molecules having a different origin. Pathogen infections in humans, sometimes leading to premature death, have been controlled to some extent in industrialized countries in the last decades due to a better comprehension of pathogen life cycle and to the design and availability of new drugs including vaccines. However, known pathogens keep on infecting people in various regions, whereas in other situations, resistance strains to existing drugs have occurred or new pathogens emerge. The needs therefore remain for the design or the identification of efficient drugs against pathogens or against their detrimental consequences in hosts and for the study of mechanisms of infection of such pathogens.

The study of pathogens can be performed both on *in vitro* or *in vivo* models. *In vitro* models, such as cell cultures, are easy to maintain at a reasonable price. However, culture cells are not always receptive to pathogens, and if they are, they do not sustain the infection for a sufficient time period enabling the study of the pathogen life cycle. Moreover, primary cultures are not differentiated enough to express markers and to secrete molecules. Finally, they are not integrated in an environment comparable to the environment offered by a live organism and consequently lack interactions with other biological systems operating *in vivo* and particularly with the immune system. As such, cellular cultures do not represent a sufficient model to study the various interactions between the pathogen and the cell in a manner, which would mimic *in vivo* interactions.

*In vivo* models often represent more relevant models than cultured cells; experiments generally are carried on mammals and particularly on mice, but also on primates. Mouse models have a lot of advantages such as being cost efficient, easy to reproduce and to manipulate. However, many pathogens cannot develop in such a host because of their restricted tropism. Besides, biological mechanisms in mice are different in many respects from those observed in human and results obtained in mice can sometimes hardly be transposed to human. To overcome such problems, experiments are performed on primates where the mechanisms of infection are more or less the same as in human, at least in higher primates such as chimpanzees. But the limited availability of these primates, the economical and ethical considerations underlying their use and the difficulty to handle them in most laboratories severely restrict their use for such purposes.

A particular group of diseases, concerned by these restrictions, are human liver diseases, such as hepatitis and malaria for which yearly cumulative mortality is close to 10 millions people. The infection caused by 3 majors pathogens, HBV (hepatitis B virus), HCV (hepatitis C virus) and *Plasmodium falciparum* for malaria, can be fought by different treatments: preventive vaccine or antiviral therapy for HBV and antiviral therapy for HCV and malaria. However, some patients do not respond to treatment and resistant strains of said pathogens are increasing both in prevalence and degree of resistance. The development of pathogen studies and new drugs is hampered by the difficulty to establish *in vitro* and *in vivo* relevant models.

The narrow host range of these pathogens prevents their efficient study in most *in vitro* models. For example, only fully functional hepatocytes of primary cultures are susceptible to *Plasmodium falciparum,* but after 1 to 3 weeks of cultivation, these cultures become refractory when phenotypic changes, *i.e.*, de-differentiation, occur (Fraslin JM. et al. 1985. Embo J. 4, 2487-2491 and Guguen-guillouzou C. et al. 1993. Cytotechnology. 11 (Suppl1 ), S3-S5). Even the most differentiated hepatoma, such as HepG2-A16 or BC2 which share 99% homology with primary hepatocytes in terms of secreted protein do not sustain *Plasmodium falciparum* maturation (Hollingdale MR. et al. 1985. Am J Trop Med Hyg 34, 261-222 and Druilhe P. et al. 1998. in Malaria: Parasite Biology, Pathogenesis and Protection, ed I.W. Sherman, 513-543).

Whereas mammal models comprising tumour cells have been described, few mammal models comprising non-infected, non-tumoral human cells are available. The need in such models or in improved *in vivo* models, reproducing to a certain extent human cell conditions is important not only for the study of infectious diseases as explained above, but also for the study of non infectious diseases, such as genetic or environmental diseases, or more generally for the study of the metabolism of compounds of human cells embedded in the animal model.

Moreover, *in vivo* animal models would be useful to perform screening activity of compounds on organisms, especially for testing effects of new drugs on live organisms. In this respect, a chimeric murine according to the invention, which comprises functional human hepatocytes, allows the study of metabolic pathways following administration of compounds. Due to differences existing in metabolic pathways between human patients and animal models usually used for screening it appears that the effects of a compound on a human biological system can sometimes be ascertained in clinical trials only. Thus, the availability of such models would enable to increase screening efficiency and thus select compounds of interest for clinical trials, in a more appropriate manner.

*In vivo* models, which have been prepared over a ten-year period, offer the potential to store human healthy and infected cells *in vivo* but still present drawbacks which harm their effective use.

For example, in order to study the stage known as late stage of *Plasmodium falciparum* cycle, *i.e.* the hepatic or exoerythrocytic (EE) stage, human hepatocytes have been transplanted in a severe combined immunodeficient (SCID) mouse, lacking both functional T and B cells (Sacci J.B. et al. 1992. Proc. Natl. Acad Sci. 89, 3701-3705). This SCID model did not reject the xenograft of human tissue, enabling the transplanted cells to maintain in their host. Subsequent intravenous injections of *P. falciparum* sporozoites led to the infection of transplanted hepatocytes as controlled by immunohistochemical staining at days 1 and 7 after the injection. The first occurrence of liver stage of *P*. *falciparum* in a mouse transplanted with human hepatocyte was obtained. However, rapidly, these results were found to be disappointing and questionable since two independent research teams had not been able, with the conditions reported in the article, to reproduce the infection, therefore contesting the maturity and functionality of the transplanted hepatocytes (Butcher GA. et al. 1993. Exp. Parasitol 77, 257-260 and Badell E. et al. 1995. Parasitology Today 11 (5), 169-171).

According to another example, in order to evaluate anti-HBV therapeutic agents, a mouse model termed "trimera" was developed (llan E. et al. 1999. Hepatology 29(2), 553-562). A normal mouse, preconditioned by lethal total body irradiation and radioprotected with SCID mouse bone marrow, was deemed to be permissive for engraftment of human tissues. The resulting model comprised three genetically disparate sources of tissues. The transplantation of *ex vivo* HBV-infected human liver fragment in such a mouse enabled HBV to replicate for a period of one month, and to generate viremia in the recipient mouse. This model enabled the infected transfected cells to maintain in the recipient and sustained the replication of the pathogen. Such a model also showed the survival of non-infected hepatocytes up to 1 month after transplantation, but not the growth of these latter.

Another strategy was adopted by the team of Ohashi et al. (Ohashi K. et al. 2000. Nat. Med. 6(3), 327-331) to create a xenotransplant model for study of human hepatitis viral infection. NOD/SCID (non obese diabetic/severe combined immunodeficiency) mice were transplanted, in the kidney capsule, with hepatocytes mixed with Matrigel. The loss of the human transplanted hepatocytes was however observed and the hypothesis was made of the absence of an essential growth factor *i.e.*, the hepatocyte growth factor (HGF). The phosphorylation of this growth factor by the addition of a specific antibody against c-met did however stabilize hepatocytes, as shown by measurement of a hepatocyte specific marker concentration *i.e.*, human alpha-1 antitrypsin (hα1AT). The authors showed that these hepatocytes had become susceptible to HBV and HDV infection and were able to support the replication of these viruses. However, though this model seemed to be appropriate to study viral infection, only viability and maintenance of transplanted hepatocytes, but no growth, could be observed. Moreover, after about 5 months following transplantation, a 35-40% decrease in hα1AT levels was observed, suggesting the persistence of probably less functional hepatocytes.

A mouse model for studying the transplantation of circulating red blood cells (RBC) and their infection by *P. falciparum* was obtained (Badell E. et al. 2000. J. Exp. Med. 192(11), 1653-1659 and Moreno A. et al. 2001. Antimicrob Agents Chemother. 45(6), 1847-1853). Mice bearing mutations affecting T and B cell functions (BXN mice) were treated with intraperitoneal injection of dichloromethylenediphosphonate (Cl₂MDP) encapsulated in liposomes and with anti-polymorphonuclear neutrophils (PMN) antibodies. This treatment enabled survival of *P. falciparum*-infected RBC and enabled the study of drugs in a chronic, stable and long-lasting parasitaemia. This model seemed to be efficient for the survival of without a nucleus and circulating cells, like RBC and nucleated protozoa such as *Plasmodium.*

Another, suitable model for HCV infection, was obtained by Mercer et al. (Mercer D.F. et al. 2001. Nat. Med. 7(8), 927-933). SCID mice (*i.e.*, mice having no functional T and B cells) were crossed with Alb-uPA transgenic mice. These latter express a transgene, the urokinase-type plasminogen activator (uPA) under the control of the albumin promoter, leading to the death of transgene-carrying hepatocytes and resulting in a growth advantage for transplanted cells devoid of said gene. The effectiveness of human hepatocyte transplantation in these crossed mice was controlled by hα1AT signal measurement. The results showed that some recipient mice had an extinction of signal around 14 weeks after transplantation, whereas a second subset maintained a strong signal beyond 30 weeks. DNA analysis confirmed that animals with sustained engraftment were homozygous for the transgene, and that the subset with unsuccessful graft was hemizygous for said transgene. This model also demonstrated that murine liver could be repopulated with human hepatocytes, but in the Alb-uPA homozygous mice only. Consequently, the homozygosity of Alb-uPA in this model was deemed to be critical to successful grafting and establishment of viral infection.

Another model showing human hepatocyte partial repopulation of murine liver was that of Dandri et al. (Dandri M. et al. 2001. Hepatology 33(4), 981-988). uPA transgenic mice were crossed with RAG-2 mice (lacking mature T and B lymphocytes), and hemizygous uPA mice were transplanted with primary human hepatocytes. A successful transplantation and partial repopulation (highest degree estimated up to 15% of mouse liver) were obtained with hepatocytes from perfused donor liver specimen. The other experiments with hepatocytes from tissues surrounding tumours or from cell solution failed to produce successful transplantation. Injection of HBV-infectious human serum in uPA/RAG-2 mice resulted in human hepatocyte infection and in presence of viral envelope protein in transplanted mouse serum. Accordingly, the transplanted hepatocytes were permissive for HBV indicating that they are functional. This model proved to be useful in the study of HBV infection when a repopulation could be obtained, i.e., with hepatocytes from healthy livers that underwent a very short ischemia time before perfusion. No transplantation using human hepatocytes obtained from a partial hepatectomy succeeded. This restriction considerably limits the human liver specimens that can be used for transplantation and accordingly the number of efficient models obtained.

The models, presented above, all face important restrictions or drawbacks limiting their use in pathogen and drug studies. Especially, the first above models were easy to produce but only enabled the survival of the implanted human cells and not their growth. The two last models allowing repopulation of hepatocytes were limited by extensive conditions: the requirement for homozygosity of the Alb-uPA transgene or the very high quality of implanted hepatocytes.

In order to allow study of pathogens having a specific tropism in human host, models have to fill in conditions that mimic to a large extent those encountered in human. Hence, it would be highly desirable to obtain a model with a degree of repopulation, which would allow cell interactions, and sufficient cell differentiation enabling regular expression of receptors and molecules. This model would be suitable for studying not only pathogen life cycle, but also for the screening of drugs or the design of compounds.

### SUMMARY OF THE INVENTION

In one aspect, the invention provides a method of making a chimeric murine model comprising:
a. obtaining an immunocompromised murine host, which carries a transgene or a genetic defect responsible for murine hepatocyte death,
b. implanting human hepatocytes therein,
c. controlling non-adaptive defences,
d. recovering a chimeric murine model harbouring settled human hepatocytes capable of maintaining, differentiating and growing.

Alternatively in such process the above steps of implanting human hepatocytes and controlling non-adaptive defences can be inverted.

The invention also provides a chimeric murine model which is an immunocompromised murine host harbouring at least one transgene or genetic defect responsible for murine hepatocyte death, which non-adaptive defences are controlled and which is implanted with human hepatocytes, said control of non-adaptive defences enable said hepatocytes to maintain, differentiate and grow.

In a particular embodiment, the chimeric murine model of the invention is also implanted with human red blood cells, in addition to human hepatocytes.

In yet another aspect, the invention provides a method for studying a hepatotropic pathogen, in a chimeric murine model according to the invention comprising:
a. infecting said chimeric murine model with a hepatotropic pathogen, in conditions enabling said pathogen to penetrate the settled human cells of the chimeric murine model,
b. observing the pathogen-generated infection in said settled cells.

The invention also relates to the use of a chimeric murine model of the invention for the testing of a compound for a potential therapeutic interest.

Another aspect of the invention is a method for screening active compounds against the infection by a hepatotropic pathogen or against its detrimental effects in a chimeric murine model according to the invention comprising:
a. infecting said chimeric murine model with a hepatotropic pathogen, in conditions enabling said pathogen to penetrate the settled human cells of the chimeric murine model;
b. administering the tested compound in conditions allowing its activity;
c. observing the effects of said compound on the pathogen-generated infection or on its detrimental effects.

A further aspect of the invention provides a method for screening the *in vivo* metabolism of xenobiotic compounds, in a chimeric murine model of the invention comprising:
a. administrating the xenobiotic compound to be tested to said chimeric murine model in conditions allowing the compound to interact with settled human hepatocytes,
b. observing its biotransformation by said settled hepatocytes.

In a particular embodiment of the invention, the human hepatocytes to be implanted can be obtained from donor liver specimens, from partial hepatectomy, from tissues surrounding tumour or can be human hepatocytes isolated from another mammal, especially murine model.

A particular murine host is an immunocompromised mouse homozygous for the SCID defect, lacking T and B cells, and homozygous or hemizygous for the Alb-uPA gene and enabling the implantation of human hepatocytes.

Particular hepatotropic pathogens for life cycle studies or drug screening are *Plasmodium* strains (*P. falciparum or P. vivax*), HBV or HCV.

### BRIEF DESCRIPTION OF THE DRAWINGS

**Fig 1**: FACS profiles
   **a)** FACS profile for macrophage depletion in untreated and treated mice. In black, cells incubated without F4/80 antibody; in red, cells incubated with F4/80 antibody.
   **b)** FACS profile for NK depletion in untreated and treated mice.
**Fig 2**: Immunohistological analysis of human hepatocytes in chimeric mouse livers.
   Six weeks after human hepatocytes implantation in untreated animals, serial liver sections were immunostained with a monoclonal antibody against human hepatocytes **(a)** or with an anti-mouse macrophage monoclonal antibody Mac2 **(b)**
   Twelve weeks after transplantation in untreated animals, serial liver sections were immunostained with an anti-human α1-antitrypsin antibody **(c)** or with an anti-mouse macrophage monoclonal antibody Mac2 **(d)**.
   Untreated **(e)** and treated **(f)** chimeric mouse livers, one month after implantation, immunostained with rabbit anti-human α1-antitrypsin antibodies.
   Untreated **(g)** and treated **(h)** chimeric mouse livers immunostained with rabbit anti-human α1-antitrypsin antibodies, 3 months after implantation.

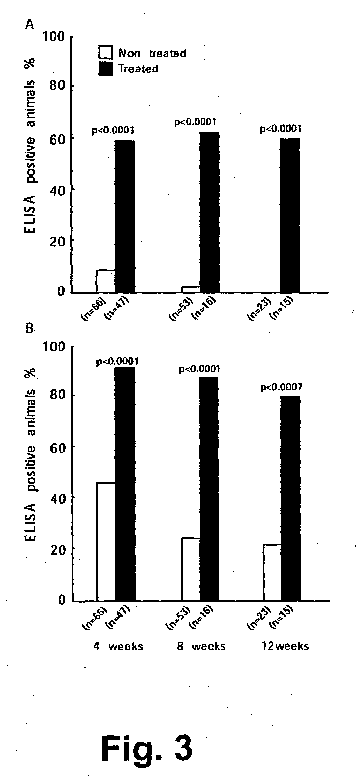

**Fig 3:** Proportion of mice positive for human albumin (HA) and α1-antitrypsin (α1AT) secretion in treated as compared to untreated implanted animals. The serum levels of HA **(a)** and human α1AT **(b)** in uPA/SCID mice were determined by a standard sandwich ELISA, and the results expressed in % positive animals.

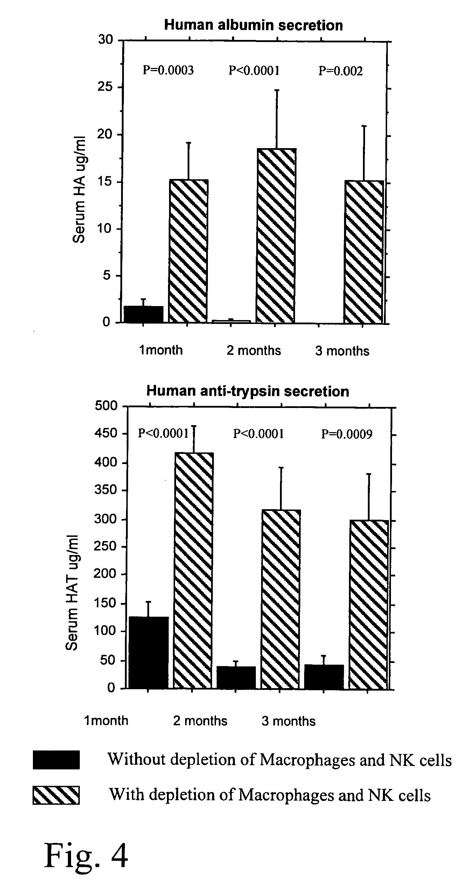

**Fig. 4**: Mean concentrations in mice sera of Human Albumin and α1 antitrypsin among treated (hatched bars) and non-treated animals (plains bars).

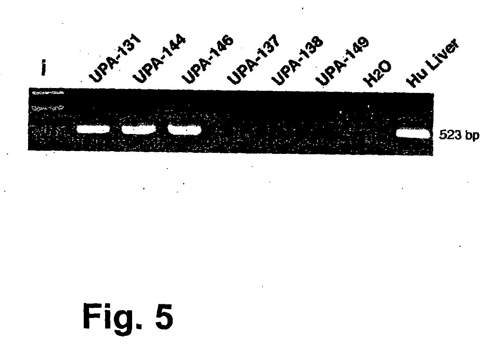

**Fig 5**: The detection of HA transcripts in RNA samples from the chimeric livers of treated (uPA 131, 144 and 146) and untreated (uPA 137, 138 and 149) animals 3 months after implantation. Human liver (Hu liver) and H₂O were used as positive and negative controls, respectively.

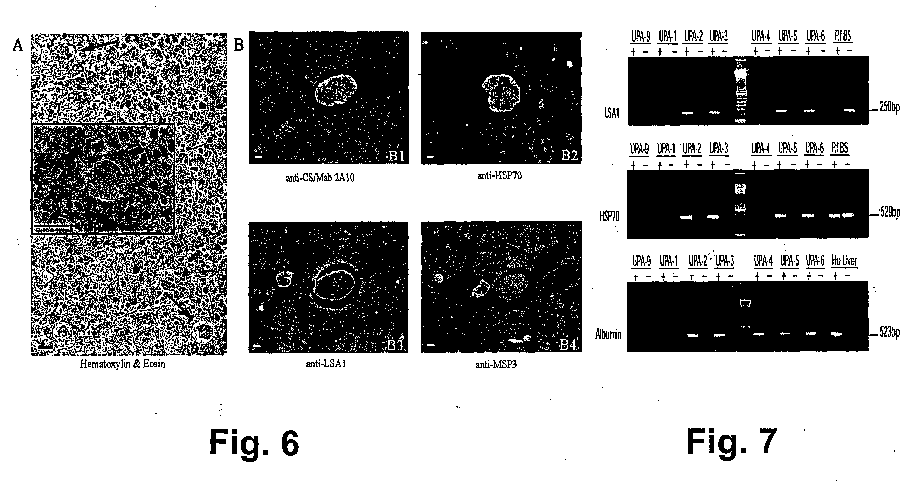

**Fig 6**: *Plasmodium falciparum* liver stage development in chimeric human liver. Bar represents 50 µm in A and 10 µm in B.
   **a)** Liver sections stained with hematoxylin-eosin. The arrows show two developing *P*. *falciparum* liver schizonts day 5 post infection; the insert shows a third schizont from the same animal.
   **b)** Liver stages of *P. falciparum* were labeled by various antibodies in IFAT: an anti-HSP70, an anti-CS protein, an anti-LSA1 and a control anti-MSP3 antibodies.
**Fig 7:** RT-PCR using primers of LSA1 (a) and HSP70 (b) on liver biopsies from implanted mice (uPA1-6) and not implanted (uPA9) and on genomic DNA from blood stages of *P. falciparum* as positive control. RT-PCR using HA primers on liver biopsies results for human albumin from implanted mice (uPA1-6) and not implanted (uPA9) and on human liver (Hu liver) as positive control. The symbol "+" and "-" refer to test (RT-PCR) and control respectively.

### DETAILED DESCRIPTION

The present invention provides a method of making a chimeric murine model comprising:
a. obtaining an immunocompromised murine host, which carries a transgene or a genetic defect responsible for murine hepatocyte death,
b. implanting human hepatocytes therein,
c. controlling non-adaptive defences,
d. recovering a chimeric murine model harbouring settled human hepatocytes capable of maintaining, differentiating and growing.

A murine host useful for the preparation of the chimeric murine model of the invention can be obtained by carrying out the following steps. First, an immunocompromised murine animal is crossed with a murine animal carrying a transgene or a genetic defect responsible for murine hepatocyte death and resulting in a survival disadvantage of the murine hepatocytes. By selective backcrosses performed according to techniques well known to those of skill in the art, a murine host, homozygous for the immunocompromised phenotype, and carrying at least one copy of the transgene or the genetic defect responsible for murine hepatocyte death is obtained.

These two features *i.e.*, immunocompromised and the transgene or the genetic defect responsible for murine hepatocyte death, are required in the same murine host, to enable implantation of human hepatocytes.

In order to prepare the chimeric murine model starting from said murine host, a step is performed to control non-adaptive defences of the host, to recover in a last step a chimeric murine model enabling the settled human hepatocytes to maintain, differentiate and grow.

An alternative in the method of generating a chimeric murine host of the invention encompasses the possibility to control non-adaptive defences before implanting human hepatocytes.

"Chimeric" as used herein relates to a murine host, which comprises cells from a xenogenic origin *i.e.,* originating from a different organism, in particular originating from a different animal species. In the invention, said cells encompass human hepatocytes. Said human hepatocytes can be provided to the murine host *i.e.*, implanted by known appropriate methods such as grafting, injection... When implanted in the host, the human hepatocytes maintain, differentiate and grow.

In a particular embodiment said implanted human hepatocytes are capable of substituting to the resident hepatocytes and/or of repopulating the model.

"Host" as used herein is a murine animal exhibiting two important features enabling xenogenic cells to settle:
- the capacity to tolerate the xenogenic human hepatic cells because of altered immunologic mechanisms resulting from genetic mutations, treatments or surgery to provide an "immunocompromised state";
- the transgene or genetic defect responsible for murine hepatocyte death leading to the survival advantage for the xenogenic human hepatocytes and facilitating their expansion or colonisation.

The genetic defect responsible for murine hepatocyte death can be located in a genetic region naturally present in the host. In such a case, the term genetic defect encompasses any genetic modification changing gene expression such as nucleotide substitution, deletion, insertion that alter the gene transcription, the mRNA stability, the protein translation or the protein stability or activity. These modifications can occur in coding or in non-coding sequences such as promoter, 5'UTR, introns or 3'UTR (untranslated region). This defect can be the result of a naturally occurring mutation or can be generated by techniques well known in the art, such as homologous recombination.

The genetic defect can also result from the insertion of a genetic construct in mammal cells. The genetic construct, such as a transgene, can be integrated in the host genome (genomic or mitochondria DNA) or can remain unintegrated, e.g., as a vector. The genetic construct can carry promoter and regulation elements and a coding sequence DNA, including cDNA, originally present or absent from the host genome.

For the invention, this genetic defect is harboured in at least one copy; it can be present in all cells or in some cell types only provided it is expressed in such a way that only resident hepatocytes of the murine host are altered, because of the different gene expression patterns. It is an object of the genetic defect to reduce the population of autologous hepatocytes in the murine host prior to implanting human hepatocytes and to enable the selective advantage in favour of these implanted human hepatocytes to take place.

As a particular transgene or genetic defect responsible for murine hepatocyte death is the Alb-uPA transgene. The Alb-uPA transgenic mouse carries urokinase gene controlled by an albumin promoter which targets urokinase overproduction to the liver resulting in a severe hypofibrinogenemic state and accelerated hepatocyte death. Randomly, individual hepatocytes spontaneously delete portion of the transgene, providing a significant replicative advantage over surrounding cells, and resulting in repopulation of the liver with nontransgenic cells (Heckel JL. et al. 1990. Cell 62(3), 447-56 and Sandgren EP. et al. 2002 Journal of hepatology 37, 422-424).

Genetic defects are all mutations, as described above, that affect the murine hepatocyte survival. As examples, other genetic defects leading to the survival advantage of implanted human hepatocytes can be envisaged:
- a FAH mutation. Mice FAH^{-/-}, because of the mutation, present a deficit in fumaryl acetoacetate hydrolase. This deficiency causes the accumulation of a hepatotoxic compound leading to a continuous process of hepatocytes lyses.
- a caspase inducible gene under an inducible promoter that can selectively destroy murine hepatocytes by activation of the promoter.

"Implanting" as used herein is the process of incorporating xenogenic cells, *e.g.* human hepatocytes, into a recipient murine host. The implantation can take place in various locations, *e.g.*, intrahepatic, intrasplenic, intraperitoneal or intraorbital and the implanted cells when settled in the chimeric murine model can circulate or to the contrary can remain at a determined location.

In a particular embodiment, implantation is realized with adult or foetal primary hepatocytes, bone marrow cells that can differentiate in hepatocytes or hepatocyte cell lines.

Before implantation, implanted hepatocytes can also have previously undergone various treatments, including genetic modifications. For example, hepatocytes can be prepared as described (Dandri M. et al. 2001. Hepatology 33, 981-988) by collagenase treatment. They can also be kept under *in vitro* conditions in cultures (*e.g.*, one to 3 weeks) before being implanted into the murine host.

Dissociated hepatocyte suspension following collagenase treatment will be implanted in the liver, the spleen or the kidney capsule, and human liver slices of various thicknesses will be for example implanted in the muscle. Implantation can also occur in the peritoneal cavity where isolated human cells are attached to collagen and other matrix sponges.

The chimeric murine model can be used to implant, human hepatocytes, and besides said human hepatocytes, enucleated cells such as red blood cells (RBC). In such a murine model where combined implantation of human hepatocytes and RBCs is provided, the present description, especially definitions disclosed with respect to human hepatocytes, can be transposed to RBCs where appropriate in consideration of the nature of the cells.

In a particular embodiment, the implanted hepatocytes are healthy cells, encompassing non-infected and non-tumoral hepatocytes.

"Non-infected" refers to hepatocytes that have not undergone, previously to the implantation, interactions with the hepatotropic pathogen which effect on said cells might be tested later in the obtained chimeric murine model. Such hepatocytes are for example obtained from a liver of a patient who has been tested negative for said pathogen or whose background enables to support that he was free from infection with said pathogen for the period of concern (*e.g.* histological and/or biological signs).

Therefore, the current invention providing a chimeric murine model with human hepatocytes, the implanted hepatocytes should not be infected by *Plasmodium* strains and/or by HVB and/or by HCV.

"Non-tumoral" refer to hepatocytes having controlled cellular proliferation and spread and having a stable karyotype, *i.e.* hepatocytes containing the same number of chromosomes after multiple divisions.

In the invention, the implanted human hepatocytes, although they are non-infected, and non-tumoral cells can nevertheless carry a mutation which effect may be studied when these hepatocytes are brought in contact with a determined compound administered to the chimeric murine model.

"Non adaptive defences" refer to cells involved in the nonspecific immunity, such as macrophages, monocytes or NK cells, in contrast to specific immunity directed by T and B lymphocytes.

"Settled" as used herein refers to human hepatocytes that are not lost after the implanting step, and that succeed in surviving and repopulating in the chimeric murine model.

The capacity of implanted hepatocytes "to maintain" as used herein refers to the capacity for these hepatocytes to survive in the host.

The capacity of hepatocytes "to differentiate" as used herein refers to hepatocytes having the capacity to reach, after the implanting step, characteristics as similar as possible to those in their original host, *e.g.*, in humans. This capacity can be determined in terms of secreted molecules (such as hα1AT for the hepatocytes), expressed surface receptors, pathogen infection, cell size or any other appropriate methods. The presence of human cell type-specific molecules expressed by the human cells can be measured, on murine sera, by well known techniques such as ELISA (enzyme-linked immunosorbent assay), Western Blot, dot blot, immunoprecipitation, direct or indirect immunostaining on histological sections using specific antibodies of implanted cell markers. The cell type-specific molecule transcripts can be detected by RT-PCR (reverse transcriptase-polymerase chain reaction) or real-time RT-PCR by using specific primers of implanted cell markers. Specific receptors can be detected by various techniques such as FACS analysis Finally, pathogen infection is controlled by detection of settled cell stage specific proteins by techniques including light microscopy, immunohistological staining on biopsies, by ELISA, PCR or RT-PCR on sera and by Western Blot, PCR or RT-PCR on cellular extracts.

The capacity of implanted hepatocytes "to grow" as used herein refers to the capacity of settled hepatocytes, not only to survive in the recipient but also to multiply in the obtained chimeric model. Growth can be measured by quantitative imaging and evaluating the percentage of cells expressing a specific cell type marker. Measurements can be made at different time points to follow the repopulation of the settled hepatocytes. The growth can also be followed by the analysis of the DNA synthesis by the incorporation of a labelled nucleotide such as BrdU.

A first advantage of the invention lies in the fact that the success of human hepatocyte implantation does not depend on the strict homozygosity of the transgene or genetic defect responsible for murine hepatocyte death, in the murine model of the invention as described in previous models. Consequently, the possibility to use murine host carrying only one copy of said transgene or genetic defect, such as hemizygous or heterozygous host for this trait, widens the scope of this model. Consequently, the number of crosses to obtain a murine host, in which an implanting step is possible, is reduced, leading to a faster and cheaper host generation.

In the invention, homozygous is defined as presenting with two copies of the same allele of a determined gene, heterozygous is defined as having two different alleles (*e.g.*, A and a) at the corresponding gene loci on homologous chromosomes for said gene and hemizygous is defined as presenting with a gene present in only one copy, not two, in an otherwise diploid cell or individual.

A second advantage of the chimeric murine model according to the invention is the possibility to implant non-infected, non-tumoral hepatocytes from various origins. The hepatocytes needed for the implanting step can come directly from donor liver, partial liver resection or tumour-surrounding tissues. The model is less sensitive than previous ones to cell conservation conditions. An attractive and outstanding feature of the present model is the possibility to perform serial implantation from chimeric murine model to chimeric murine model, allowing faster and more efficient subsequent implantations.

The inventors have determined that controlling non-adaptive defences of the host is one of the parameters enabling the implanted hepatocytes to settle, differentiate and grow in the chimeric murine model.

The efficiency of the control of non-adaptive defences can be checked by various techniques, such as FACS analysis. The main actors of the non-adaptive defences are macrophages and NK cells for which a strong reduction or depletion is expected after immunomodulation treatment according to the invention.

"Macrophage depletion" as used herein is the process of reducing in a large amount the circulating and tissue macrophages. A convenient range of remaining macrophages after treatment is 0% to 50%. A preferred range of remaining macrophages is 0% to 20%.

Macrophage number can be reduced by administrating in the host, antagonists of macrophages, such as toxic substances, like Cl₂MDP, or antibodies altering macrophage development or function and finally killing them. The administration of antagonists is performed by well-known techniques, including the use of liposomes.

"NK depletion" as used herein is the process of reducing NK cells after treatment. A convenient range of remaining NK after treatment is 0% to 50%. A preferred range of remaining NK is 0% to 20%.

The administration of NK antagonists or substances altering their function and development is made by well-known techniques using vectors including liposomes.

In a particular embodiment, the macrophage depletion is realized by injecting liposomes containing Cl₂MDP according to the technique of Van Rooijen et al. (Van Rooijen N. 1989. J. Immunol. Methods 124, 1-6). The liposome size can range from 0.5 to 7 µm to be ingested by macrophages, resulting in their killing.

Possible alternatives for reducing the macrophage population are either the administration of another toxic, the cis-platinium or the use of the irradiation. However, these techniques are less efficient than the administration of liposomes containing Cl₂MDP.

The NK cells depletion is preferably realized by injecting an anti-NK antibody, such as the anti-Tim β1 mouse monoclonal antibody, binding to the IL-2R β chain.

A particular protocol of macrophage depletion is the injection of Cl₂MDP embedded in liposomes, at 4-day interval, starting two days after implantation. Such liposomes fully clear macrophages from peritoneum, liver, spleen and kidney. Monocytes from the bone marrow colonise the liver after the clearance of all Kupfer cells and transform into very active large macrophages and new Kupfer cells. These are, again, destroyed by the next injection of liposomes.

In a particular embodiment for NK depletion, anti-NK antibodies are injected at monthly interval, starting two days after implantation. The interval between injection depend on the half-life of the antibody directed to NK cells, and can be from 2 to 4 weeks in order to keep NK cells continuously low.

Particular immunocompromised models used to generate a host are SCID mouse (severe combined immunodeficiency), SCID/Nod mouse (severe combined immunodeficiency/non obese diabetic) or mouse with altered lymphocyte lineages such as BXN (NIHIII or Beige Xid Nude), RAG, RAG2 and RAG-γC.

In a particular embodiment of the invention, the mouse host is obtained by:
a. crossing a homozygous SCID mouse with a homozygous Alb-uPA transgenic mouse;
b. making selective backcrosses;
c. recovering a homozygous SCID, hemizygous Alb-uPA mouse host.

The present invention provides also a chimeric murine model which is an immunocompromised murine host harbouring at least one transgene or genetic defect responsible for murine hepatocyte death, which non-adaptive defences are controlled and which is implanted with human hepatocytes, said control of non-adaptive defences enable said hepatocytes to maintain, differentiate and grow.

An advantage of the chimeric murine model of the invention is not only the maintenance of the settled human hepatocytes, but also their growth and differentiation.

The growth and differentiation of the settled human hepatocytes of the chimeric murine model of the invention can be checked by various features, such as the presence of cell surface receptors, the secretion of proteins specific of the implanted hepatocytes, the cell size or the receptivity to pathogens.

In a particular embodiment of the invention, the model enables the settled human hepatocytes to secrete specific proteins characterizing their differentiation and growth, for several months especially for at least 3 months. Specific proteins of the implanted hepatocytes, such as albumin, can be used as markers to follow the differentiation state as well as the repopulation.

The hypothesis that implanted human hepatocytes acquired a differentiation state similar to that observed in *in vivo* human conditions, can be tested by the stringent requirements of some pathogens to infect differentiated hepatocytes to realize their development.

Another advantage of the murine model of the invention is the receptivity of settled hepatocytes for pathogens having a restricted hepatotropism.

"Receptive" as used herein refers to the capacity of settled hepatocytes to sustain an infection similar to that observed in humans. Therefore, pathogens penetrate settled hepatocytes and realize their replication and maturation.

According to another embodiment of the invention the model is suitable for *in vivo* study of metabolism of administered compounds and especially drugs, in said settled hepatocytes.

As a particular embodiment, the murine host is a homozygous SCID, hemizygous Alb-uPA mouse, comprising implanted human hepatocytes. In this chimeric murine model, settled cells are receptive to hepatotropic pathogens, such as HBV, HCV or *Plasmodium* strains, *e.g. P. falciparum* or *P. vivax.*

Another particular chimeric murine model is a chimeric murine animal, comprising implanted human hepatocytes and human red blood cells (RBC). In this particular model, it might be possible to follow-up the life cycle of *Plasmodium falciparum* in the same animal model. The pathogen, following its replication in human liver cells can invade implanted RBCs and develop to schizont stage in those RBCs.

The chimeric murine model of the present invention allows the study of restricted-tropism pathogens, for which no permissive line exists or for which permissive models are not fully satisfactory. The chimeric murine model of the present invention contains at least settled hepatocytes that are differentiated and are able to sustain a pathogen infection for several weeks. Because of the duration of the settled hepatocyte survival, their repopulation and their high number in the chimeric murine model, pathogen life cycle can be intensively studied including when said infected hepatocytes are put in contact with candidate drug compounds.

The invention also provides a method for studying a hepatotropic pathogen, in a chimeric murine model according to the invention comprising:
a. infecting said chimeric murine model with a hepatotropic pathogen, in conditions enabling said pathogen to penetrate the settled human cells of the chimeric murine model,
b. observing the pathogen-generated infection in said settled cells.

The first step is the introduction in the chimeric murine model of a pathogen in conditions in which it can interact with settled hepatocytes and especially can penetrate them. The introduction of the pathogen can be achieved by various ways, including intravenous or intracutaneous injections.

In a second step, the infection of said settled hepatocytes is monitored by well known methods including, but not limited to, light microscopy, immunofluorescence antibody test (IFAT) using pathogen specific antibodies, PCR (qualitative or quantitative) or RT-PCR using primers for a pathogen specific gene, ELISA or immunoprecipitation. When a pathogen has a life cycle with different forms infecting various organs or various species, antibodies and primers are chosen to be specific to proteins of each form, and in the invention, specific of the proteins of the settled cell form. All the results obtained to identify the pathogen infection, for example the number of pathogens calculated by light microscopy, the staining observed by IFAT and the mRNA transcripts detected in the settled cells of the chimeric murine model, are compared to a control model infected by the same pathogen but without cell implantation.

In a particular embodiment, the chimeric murine model tested is a mouse generated from a homozygous SCID, hemizygous Alb-uPA mouse host, in which hepatocytes are implanted.

Another particular chimeric murine model is a murine host according to the invention implanted with both human hepatocytes and human red blood cells (RBC).

Particular hepatotropic pathogens are HBV (hepatitis B virus), HCV (hepatitis C virus) or *Plasmodium* strains including *P. falciparum* and *P vivax.*

The monitoring of the hepatocyte infection by *P. falciparum* can be performed by testing pathogen proteins, specific for hepatocytes and/or erythrocytes, such as the circumsporozoite protein (sporozoite), the MSP3 protein (erythrocytes), the HSP70 and MSP1 proteins (hepatocytes and erythrocytes) and the LSA1 protein (hepatocytes).

The monitoring of the hepatocyte infection by HCV can be performed by measuring the presence or absence of the viral RNA sequence (qualitative PCR) or the viral load (quantitative PCR).

The monitoring of the hepatocyte infection by HBV can be performed by quantifying a viral envelop protein HbsAg with the ELISA technique.

The invention provides also the use of of a chimeric murine model of the invention for the testing of a compound for a potential therapeutic interest, towards the infection or its consequences.

The chimeric murine model can also be useful for the screening of drugs capable of altering the life cycle of pathogens. Owing to the capacity of the chimeric murine model to sustain a pathogen infection for several weeks, the effects of a drug administration on the infection can be observed and its efficacy can be evaluated. The invention provides a method for screening active compounds against the infection by hepatotropic pathogen or against its detrimental effects in a chimeric murine model of the invention comprising:
a. infecting said chimeric murine model with a hepatotropic pathogen, in conditions enabling said pathogen to penetrate the settled human cells of the chimeric murine model;
b. administering the tested compound in conditions allowing its activity;
c. observing the effects of said compound on the pathogen-generated infection or on its detrimental effects.

In a first step, settled hepatocytes are infected according to the method described above.

Then, the drug is administered to the chimeric murine in conditions in which the drug keeps, modifies or acquires its activity. The interactions with the settled hepatocytes as well as the host environment could be determined with respect to the activity of the drug. "A drug" as defined herein is any substance presented for treating, curing or preventing disease in human beings or in animals, including prodrugs.

The drug can be administered under any appropriate forms including systemic or local routes.

Several drugs (at least two) can be administrated together, alternatively or with specific protocols to show a possible synergy, redundancy or antagonism.

The drug can be administrated by a lot of well known routes, including taken by mouth (orally), given by injection into a vein (intravenously), into a muscle (intramuscularly), beneath the skin (subcutaneously) or placed under the tongue (sublingually), inserted in the rectum (rectally) or vagina (vaginally), instilled in the eye (by the ocular route); sprayed into the nose and absorbed through the nasal membranes (nasally); breathed into the lungs (by inhalation) or applied to the skin (cutaneously).

Finally, the effects of the drug on the pathogen infection or on its detrimental effects can be monitored. Various techniques can be used to observe the effects of the administrated drug, including light microscopy, immunofluorescence antibody test (IFAT), RT-PCR, PCR (qualitative or quantitative), or ELISA. The infection are followed at different time points and various factors can be calculated: drug half-life, minimal effective dosage for infection elimination or reduction, drug dosage based on age, on body weight or on body surface area, the most efficient place or route for drug administration, the combination therapy efficiency. Other factors that can be observed could be the biological activity of the drug metabolites produced by settled human hepatocytes as well as the toxicity on the various organs of the murine model of these human specific metabolites.

The invention also provides a method for screening the *in vivo* metabolism of xenobiotic compounds, in a chimeric murine model of the invention comprising:
a. administrating the xenobiotic compound to be tested to said chimeric murine model in conditions allowing the compound to interact with settled human hepatocytes,
b. observing its biotransformation by said settled hepatocytes.

This subset of human hepatocytes can be exploited to follow the biotransformation of a xenobiotic, after its injection into the chimeric murine model. "Xenobiotic" as used herein refers to a chemical substance (or more generally, a chemical mix) that is not a normal component of the organism in which it is exposed to. Xenobiotics include most drugs (others than those compounds which naturally occur in the organism) and prodrugs, as well as other foreign substances.

The xenobiotic is administrated into the chimeric murine model according to all routes and all forms cited above for the drug. One condition in the administration is that the xenobiotic can interact with the settled hepatocytes.

The measurement of the level of the metabolites (degradation products), including intermediates and final products, enables to track the xenobiotic metabolism kinetics, including its half-life. The model enables also to observe the effects of the compound on the settled cells, to evaluate the doses at which the effects appear. Finally, it is possible to study the potential interactions between reactive metabolites and cellular macromolecules.

This model is important for prodrugs, *i.e.*, any compound that undergoes biotransformation prior to exhibiting its pharmacologic effects. Indeed, the human hepatocytes can produce metabolites, especially the active form of the drug, from the prodrugs, whereas the murine hepatocytes cannot do it.

A particular murine model tested is a chimeric mouse model generated from a homozygous SCID, hemizygous Alb-uPA mouse host, in which hepatocytes are implanted. In this chimeric model, one could monitor the cytotoxic effects of the drug on the liver, e.g., by measuring the circulating hepatic transaminases and by analysing the liver histology with optical microscopy techniques.

The invention also provides technical platforms comprising at least the chimeric murine model of the invention such as:
- a technical platform, useful to identify new compounds useful to treat mammal infections provoked by a hepatotropic pathogen, characterized in that it comprises at least a chimeric murine model as defined above and appropriate means to detect or to observe the effects of said compounds on a pathogen-generated infection of said murine model.
- a technical platform, useful for screening the *in vivo* metabolism of xenobiotic compounds characterized in that it comprises at least a chimeric murine model according to the invention and appropriate means to observe the biotransformation of said compounds by implanted human hepatocytes in said murine model.

### EXAMPLES

Examples are given to illustrate but not to limit the invention.

### Example 1: generation of a chimeric mouse model

### Animals

Homozygous Alb-uPA transgenic mice and homozygous SCID mice were purchased from Jackson Laboratories (Maine, USA) and IFFA-CREDO, respectively. Animal were housed and maintained under specific pathogen-free conditions. To generate Alb-uPA/SCID mice, we crossed Alb-uPA and SCID mouse lines, and through selective backcrossed bred trait to homozygosis. uPA transgenic mice were identified by PCR of mouse-tail DNA with the following primers: p1 :5'-ATTCTGGAGGACCGCTTATCTGT-3'; p2: 5'-CTTGAACCCAGGAGGCGGAGATT-3'. Only hemizygous uPA mice were used as host for implantation and infection experiments.

Homozygosity of the SCID trait was confirmed by quantification of total serum IgG and IgM using a sandwich ELISA. Only mouse with total Ig < 50 µg/ml were used for experiments.

### Isolation of human hepatocytes

Primary human hepatocytes were isolated as described elsewhere (Guguen-Guillouzo C. et al. 1986. Prog Liver Dis 8, 33-50) from the healthy liver tissue of surgical liver biopsies specimens (approx. 20-25 cm³) obtained with informed consent from patients who underwent therapeutic partial hepatectomy for liver metastasis and benign hepatic tumor. Subjects with viral infections (HCV, HBV, HIV), cirrhosis and primary hepatic carcinoma were excluded.

Resected liver lobes were cut at a distance of at least 3 cm from the metastasis. Human hepatocytes were isolated by a two-step perfusion technique. Hepatocyte viability obtained by this method depends on 3 important factors which are the temperature: 37°C, pH: 7.6 and perfusate flow rate: 16-20ml according to the rejection size. Perfusion began with a HEPES buffer, without calcium, at a temperature, a pH and a perfusate flow rate as indicated above. Hepatocytes were then isolated with a perfusion of 200 ml of HEPES buffer supplemented in Collagenase H 0.05% (Roche Molecular Biochemicals) and CaCl₂ 5 mM, and separated from non-parenchymatous cells by Percoll fractionation, as previously described (Giannini C. et al. 2003. Hepatology 38, 114-122; Guguen-Guillouzo C. et al. 1993. Cytotechnology 11 Suppl, S3-5; Guguen-Guillouzo C. et al. 1982. Cell Biol Int Rep 6(6), 625-8). Viable cells were determined by trypan blue exclusion.

### Implantation of human hepatocytes

For hepatocyte implantation, mouse hosts were anesthetized and a laparotomy realized. A number of 5-6 x 10⁵ viable human hepatocytes were injected into the splenic parenchyma of 10-14 days old recipients, as previously described (Giannini C. et al. 2003. Hepatology 38, 114-122).

### Macrophage and NK depletion treatment

Mice were injected intraperitonealy at 4-day interval starting 2 days after implantation with 50µl of a solution at 50% hematocrit of encapsulated-dichloro-methylene-diphosphonate (Cl₂MDP) liposomes. For NK cells depletion, these mice received at monthly interval starting at day 2 post-implantation 1 mg of purified anti-Tim-β1 (anti-IL-2 Rβ chain) mouse monoclonal antibody.

### Example 2: macrophage and NK detection

### FACS analysis

The depletion of macrophages and NK, following treatment, were investigated by FACS analysis.

For macrophage analysis, 50 to 150 µl of blood (collected by retro-orbital sampling) from treated and untreated mice were diluted in phosphate buffer. Cells are washed in this buffer twice by centrifugation at 1500 rpm for 10 minutes at 37 °C. The pellet is resuspended in a solution containing a F4/80 antibody and incubated for 30 minutes at room temperature. Cells are then washed in a Facs buffer (Becton Dickinson). The features of the F4/80 are the following: rat anti-mouse F4/80 antigen, conjugated to Fluorescein Isothiocyanate Isomer I (FITC) from Serotec.

For NK cell analysis, spleens from treated and untreated mice were taken and splenocytes were obtained after dilaceration (Cell strainer, Becton Dickinson). Cell suspension is then washed twice by centrifugation at 1500 rpm in RPMI 1650 medium (Life Sciences). Cells are then incubated with DX5 and anti-CD3 (clone17A2) antibodies, respectively coupled to FITC and Phycoerythrin for 30 minutes at room temperature. Cells are then washed and resuspended in Facs buffer (Becton Dickinson). The used antibodies have the following features:
- DX5 antibody: rat antibody, specific of CD49b/Pan-NK cells (Becton Dickinson),
- 17A2 antibody: a rat anti-mouse CD3 (T-cell receptor-associated CD3 complex), conjugated with FITC (Becton Dickinson).

### Immunostaining

Mouse liver biopsies fixed in 10% formalin were embedded in paraffin. To detect macrophages, sections were immunostained with a rat anti-mouse macrophage monoclonal antibody Mac2 (culture supernatant diluted to1:4; TIB 166, ATCC Manhasset, VA), 1 hour at room temperature. Horseradish peroxidase labelled second anti-rat antibodies (1:500 dilution) were incubated for 30 min followed by reaction with diaminobenzidine/hydrogen peroxidase as chromogen-substrate. Endoperoxydase activity was blocked by first treating the slides with 0.3% H₂O₂ in PBS for 5 min at room temperature. After immunochemistry, sections were counterstained with Meyer's hematoxylin (DAKO).

### Example 3: serum human albumin and α1 antitrypsin detection

The levels of human albumin and human α1-antitrypsin (hα1AT) in uPA/SCID mouse sera were quantified by a standard sandwich ELISA. Mouse anti human albumin (0.1 µg/ml, clone HSA-9, Sigma, St Louis, MO) and goat anti-hα1AT (11µg/ml; Rockland, Gilbertsville, PA) were used as antigen-specific capture antibodies. Following overnight coating, non specific binding was blocked by incubation with 1 % bovine serum albumin for 1 hour at 37°C. After washing, 75 µl/well of 1:10 diluted mouse sera in PBS, were added and incubated overnight at 4°C. HRP-conjugated rabbit anti-human albumin (0.16 µg/ml; Sigma Chemical Co.) and HRP-conjugated rabbit anti-hα1AT (2µg/ml) were used as antigen-specific indicator antibodies. The chromogen and the substrate were used according to the manufacturer indications (Sigma Chemical Co.). Absorbance values (405 nm) were converted to concentrations in µg/ml by comparison with a standard curve performed by using serial dilutions of defined amounts of purified human albumin and hα1AT (Sigma Chemical Co.). The ELISA result was considered as positive for the detection of human proteins (HA, hα1AT) when the OD value was higher than the OD means + 2 fold the standard deviation of 14 non-implanted Alb-uPA/SCID mice. Qualitative comparisons were done using the chi 2 test and Fischer exact test. P values of less than 0.05 were considered as significant.

### Example 4: albumin transcript detection

RT-PCR was used for human albumin transcript detection. Total RNAs were isolated from human-mouse chimeric hepatocytes, murine and human liver tissues (and human cells) using RNeasy Protect Midi kit (Qiagen S.A., France) according to the manufacturer indication. Samples were quantitated using a spectrophotometer, and equal amounts of RNA from all samples were subjected to cDNA synthesis using random primers. Human specific albumin primers, 5'-CATTAGCTGCTGATTTTGTTGAAAG-3' and 5'-TGTGCAGCATTTTGTGACTCTG-3' were used to detect human albumin transcripts. PCR Conditions were 95°C for 5 min; 94°C for 30s, 60°C for 1 min, and 72°C for 1 min for 40 cycles, with a final extension at 72° C for 5 min. Twenty µl of final PCR product were analyzed by electrophoresis (2% agarose gel with Ethidium Bromide) and PCR product bands (523 bp) were visualized under UV trans-illumination.

### Example 5: immunohistological staining alpha-1 antitrypsin protein detection

Mouse liver biopsies fixed in 10% formalin were embedded in paraffin. To detect human hepatocytes, 5µm sections pre-treated with Dako Biotin Blocking System (Dako, Glostrup, Denmark) were immunostained with a monoclonal antibody against human hepatocytes (Clone OCH1E5, 1:20 dilution; Dako, Glostrup, Denmark), using the Dako ARK system. Similarly, 5µm sections were immunostained with rabbit anti-human α1-antitrypsin antibodies (Dako, Glostrup, Denmark) diluted (1:2000) for 1 hour at room temperature. Slides were developed using a secondary anti-rabbit antibody conjugated with horseradish peroxidase (EN-VISION-kit; Dako) following the manufacturer's instructions.

### Example 6: liver repopulation estimation

Quantitative imaging was performed on the liver biopsies of three untreated and treated animals obtained at one and three months after implantation. For each animal three different sections were immunostained with rabbit anti-halAT and counterstained with Meyer's hematoxylin as described above. Adobe PhotoShop 6.02 software was then use to select and count the pixels corresponding to the total liver section (Meyer's hematoxylin staining) or to human hepatocyte clusters (anti-hα1AT reactivity). The percentage of liver repopulation by human hepatocytes was expressed as the cell surface stained for hα1AT divided by the total cells surface of the tissue section. Qualitative comparisons were done using Fischer exact test. P values of less than 0.05 were considered significant.

### Example 7: effective depletion of NK cells and macrophages

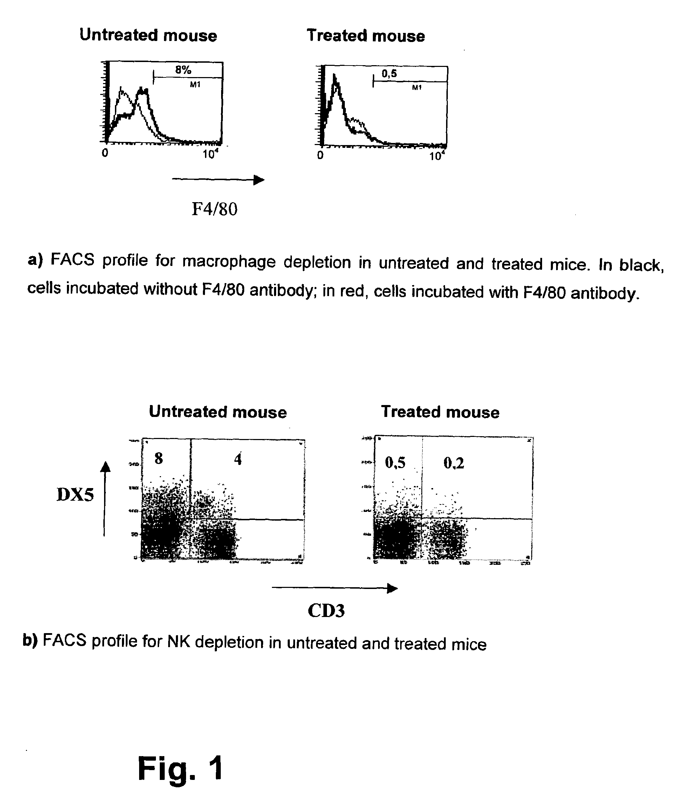

FACS analysis demonstrated, using F4/80 marker, that macrophage depletion in mice was effective since two days after the first liposome injection, macrophages represent 0.5% of lymphocyte cells in treated mice versus 8% in untreated mice (Fig1a). In addition, a successful depletion of NK cells was reached, since after Timβ-1 injection, DX5⁺CD3⁻ NK represent 0.5% of spleen lymphocyte cells in treated mice versus 8% in untreated mice (Fig 1b).

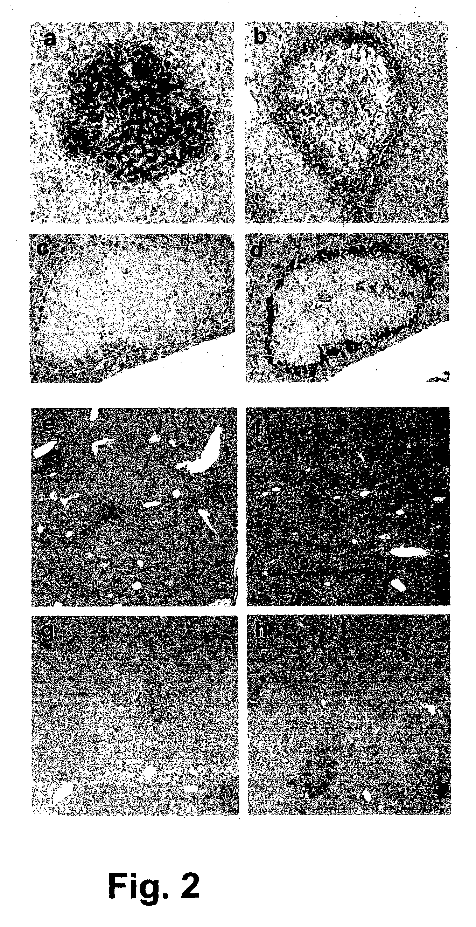

These depletion results were confirmed by histochemical analysis of human/mouse chimeric liver sections from animals implanted 6 weeks earlier. These investigations showed that the intrasplenically injected-hepatocytes had indeed migrated to the liver parenchyma and formed clusters as revealed with positive label of a human hepatocyte marker (Fig 2a). In untreated mice, these clusters were surrounded by numerous mononuclear cells (labeling with Mac2), which consisted mainly of macrophages (Fig 2b).

### Example 8: human albumin and α1 antitrypsin secretion

The ELISA analyses performed on sera of treated and untreated implanted hosts were shown on Figure 3a (human albumin) and 3b (human α1AT). Over a 3-month period after implantation, the number of positive mice for human albumin and α1 antitrypsin secretion was dramatically improved by the treatment. In untreated animals, the number of positive mice for HA and hα1AT secretion, decreased over time during the follow-up (from month 1 to month 3: 13.6 to 0%, and 45.5 to 21.7%, respectively). In contrast, in treated animals, the number of mice positive for HA and hα1AT secretion was markedly higher at one month and, above all, remained high over time during the follow-up. At three months, 60.7±1.6% and 86.3±5.8% of mice secreted HA and hα1AT respectively. This first results showed a clear correlation between the treatment and the human albumin and α1 antitrypsin secretion.

### Example 9: histochemical hα1AT protein expression

Immunohistochemical investigations at 3 months post-implantation in untreated animals, revealed that human hepatocyte clusters surrounded by macrophage became negative for hα1AT (Fig. 2c-d), suggesting that human hepatocytes were no longer functional, in agreement with the lack of detectable human serum albumin at that time (Figure 4). These data confirmed the strong relationship between the number of surrounding macrophages and the hepatocyte functionality.

Further albumin and α1 antitrypsin analyses were performed on untreated and treated animals at 1, 2 and 3 months (Figure 4). A strong reduction in macrophage numbers in the treated animals was associated with both an increase in the number of human hepatocyte clusters and the number of hepatocytes per cluster according to untreated animals, both at one month (Fig. 2e-f) and at 3 months (Fig. 2g-h).

These results taken together confirmed that the macrophage and NK depletion is necessary to the implanted hepatocytes to become fully functional, *i.e.* to secrete both human albumin and α1 antirypsin in mouse serum (Figure 4).

### Example 10: repopulation of the chimeric liver

Quantitative imaging on liver sections from implanted animals revealed that the percentage of human hepatocyte liver repopulation (expressed as the cell surface percentage, stained for hα1AT) was higher in treated as compared to untreated animals. The repopulation was of 17.7±2.1% in treated animals versus 3.1±1.5 % in untreated animals at one month (*p*=0.0006), and 26.9±4.2% versus 6.5±2.4% at 3 months (*p*=0.002). Individual data about 12 mice (6 treated and 6 untreated) are given in Table
1. This experiment showed that the implanted cells not only survive in their host, but also can multiply and repopulate the chimeric liver.

### Example 11: HA transcript expression

HA transcripts were detectable by RT-PCR in RNA samples from the chimeric livers of treated animals but not from untreated animals, 3 months after implantation (Figure 5).

Taken together, these results indicate that innate immune mechanisms deeply influence the survival and function of implanted human hepatocytes in the chimeric mouse model. This confirms the highly differentiated status of the settled cells obtained, following treatment. To demonstrate unequivocally that the implanted human hepatocytes were indeed functional, we took advantage of the stringent requirement of *P.falciparum* liver stages for fully differentiated human hepatocytes.

### Example 12: P.falciparum infection challenges

*Plasmodium falciparum* sporozoites (strain NF54) were obtained from infected *Anopheles stephensi* mosquitoes salivary glands on day 14-16 after membrane feeding on gametocytes from NF54 cultured gametocytes as previously described (Ponnudurai T. et al. 1986. Parasitology 93(Pt2), 263-74; Sinden RE. et al. 1979. Parasitology 79(2), 277-96; Sinden RE. et al. 1984. Parasitology 88(Pt2), 239-47; Ozaki I.S. et al. 1984. J Parasitol 70, 831-833).

A number of 100,000 to 1,000,000 *P. falciparum* sporozoites, the invasive forms injected by the mosquito, were intravenously injected in the retro-orbital sinus 3 to 6 weeks after human hepatocytes implantation. A total of 14 mice implanted with hepatocytes from 4 distinct donors were challenged by parasites from four mosquito batches and liver biopsies taken 5 days later. *P. falciparum* liver forms were detected by light microscopy of hematoxylin-eosin stained sections. Non implanted mice, or implanted mice negative for human albumin served as controls.

### Example 13: detection of the hepatocyte infection by P. falciparum Immunohistological staining

For protein detection, several antibodies against pathogen specific-stage proteins were used.
- an anti-circumsporozoite protein mouse monoclonal antibody, Mab2A10, specific of the circumsporozoite (CS) protein, a molecule expressed in sporozoites and carried over in liver stages (Atkinson C.T. et al. 1989. Am J Trop Med Hyg 40, 131-140; , Suhrbier A. et al. 1988. Eur J Cell Biol 46, 25-30; Wirtz R.A. et al. 1987. Bull World Health Organ 65, 39-45).
- a polyclonal antibody (anti-PfHSP70-1), obtained from immunized mice a heat shock protein expressed in both liver and blood stages; these antibodies recognize parasite-specific epitopes of the HSP70 of *Plasmodium* and do not cross-react with HSP70 from mouse or human (Renia L. et al. 1990. Eur J Immunol 20, 1445-1449; Motard A. et al. 1995. Int Immunol 7, 147-150).
- an anti-MSP1 human affinity purified antibodies, specific of MSP1, expressed in both liver and blood stages. Anti MSP1 antibodies have been affinity purified on the MSP1-19 recombinant protein covering the C-terminus part of the MSP1 molecule (Renia L. et al. 1990. Eur J Immunol 20, 1445-1449; Motard A. et al. 1995. Int Immunol 7, 147-150).
- an antibody, obtained from an LSA1 immunized chimpanzee, specific of LSA1, the only antigen known to be solely expressed in liver stages. The LSA1 antibodies are directed to several regions of the LSA1 molecule and particularly the peptides LSA1-R and LSA1-J (Guerin-Marchand C. et al. 1987. Nature 329, 164-167; Fidock D.A. et al. 1994. Nature 161, 126).
- an antibody specific of MSP3, an antigen expressed only in blood stages. Anti-MSP3 affinity purified antibodies are directed to peptide MSP3-b, peptide MSP3-c and peptide MSP3-d (Brahimi K. et al. 1993. J Immunol Methods 162, 69-75).

All sera were used at a 1:100 dilution, except for Mab2A10, used at 1:500. Moreover, a serum from a malaria non-exposed individual and a naïve mouse serum served as negative controls. Secondary, fluorescent Alexa 488-labelled anti mouse or human antibodies (Molecular Probes®) were diluted 1:600.

Mouse liver biopsies fixed in 10% formalin were embedded in paraffin. Immunohistological detection of parasites was performed on day 5 post infection with *P. falciparum* sporozoites. Liver biopsies were taken and immediately processed for histological analysis. Hematoxylin/eosin (HE) staining and IFAT were performed on serial 5 µm sections.

### RT-PCR

For parasite transcript detection, two transcript genes were tested: LSA1, a gene expressed only in liver stages and HSP70, a heat shock protein gene, expressed in both liver and blood stages.

Total RNAs were extracted, as described above, from a) implanted and non implanted infected mouse liver tissue taken 5 days following sporozoites inoculation, b) from human non-infected liver tissue and c) from *P. falciparum* erythrocytic stages, and treated with RNase free DNase. Reverse transcription was carried out under a final volume of 20 µl using 4 µg of total RNA and 200 units of M-MLV Reverse Transcriptase (Invitrogen) in the presence of specific LSA1 and HSP70-1 primers (from either LSA1 or HSP70-1 *P. falciparum* genes). Nested RT-PCR was carried out using 2µl of the RT reaction, in the presence of 125 µM dNTPs, 125 nM of primers (LSA1-N and HSP70-N), and 2 units of Taq polymerase (Qiagen). Direct PCR on RNA extracts was performed as a control to rule out genomic DNA contamination in RNA preparations. The parasite specific primers used for the reverse transcription and the nested RT-PCR analysis are as follows:

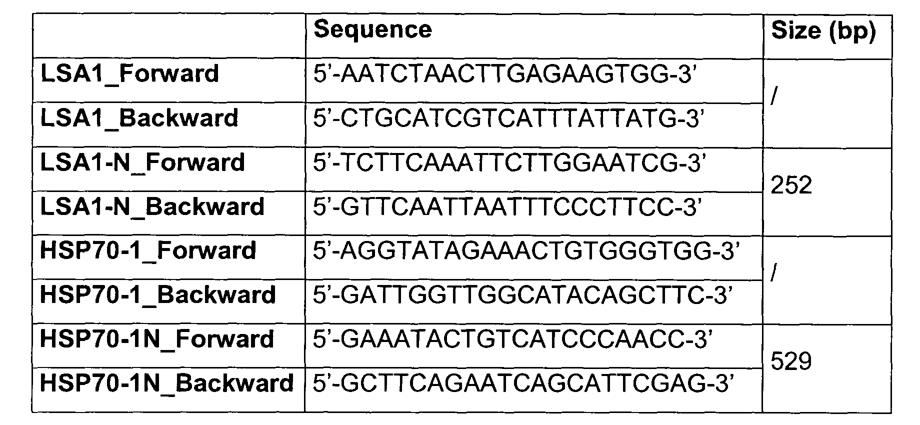

### Example 14: Plasmodium falciparum infection in hepatocytes

By light microscopy analysis, *P. falciparum* liver forms were found to be unexpectedly abundant (Fig. 6a) and this high number apparently correlated with serum albumin levels (ie. the largest number of parasites was seen in mice having a serum albumin level of about 100 µg/ml).

Morphological indications were confirmed by immunofluorescence labeling with parasite-specific reagents. The Mab2A10 antibody, specific of the circumsporozoite (CS) protein, specifically labeled the same liver form (in serial sections), as others in sections from different animals (Fig. 6b). Antibodies directed against either HSP70 or MSP1, both expressed in liver and blood stages, also reacted specifically with *P. falciparum* liver forms produced by sporozoite inoculation (Fig. 6b). Finally, antibody specific of LSA1, the only antigen known to be solely expressed in liver stages, equally labelled the liver forms (Fig. 6b).

In contrast, several control antibodies (anti-MSP3 or unrelated antibodies) yielded negative results on serial cuts from positive animals. Conversely, liver form-specific antibodies were negative on liver sections from non-implanted though infected control mice (over 100 sections screened).

The results obtained both by light microscopy and immunofluorescence staining, were further confirmed by RT-PCR, using primers specific for parasite or human genes. Human albumin transcripts were detected in 7 of 7 albumin secreting mice, in 3 of 7 implanted mice without detectable HA in the serum and not in 3 non implanted control mice 5 days after sporozoite inoculation (Figure 7).

Nested RT-PCR, performed using parasite-specific primers derived from LSA1 and HSP70 sequences, yielded homogeneous results in agreement with the above findings. Both corresponding messenger RNAs were detected in 7/10 mice in which albumin transcripts were detected (Fig. 7).

These experiments showed the high degree of maturation of the liver settled cells, as confirmed by their size, reaching 50-60 µm at day 5, *i.e.* as large as that obtained in humans (Shortt, HE. et al. 1948. Nature 161, 126 and Shortt HE. et al. 1951. Trans R Soc Trop Med Hyg 44, 405-419) or higher primates such as chimpanzees (Bray RS. 1962. Am J Trop Med Hyg 7, 20-24 and Meis JF. et al. 1990. Exp Parasitol 70, 1-11), and nearly twice as large as that obtained under *in vitro* conditions in primary human hepatocyte cultures (Meis JF. et al. 1986. Cell Tissue Res 224, 345-350; Mazier D. et al. 1985. Science 277, 440-442; Smith JE. et al. 1984. Lancet 2, 757-758). These settled cells are receptive to *P. falciparum,* in which the pathogen can develop at densities similar to those obtained with rodent parasites (Wery M. 1968. Ann Soc Belg Med Trop 48, 11-137), despite the small number of human hepatocytes implanted and the fact that parasites were inoculated in the peripheral blood.

**Table 1:**

| Mouse repopulation in treated and untreated mice, 1 month and 3 months after implantation. For each mouse, 3 values corresponding to 3 independent measures and a mean of these 3 measures are given. | | | | | | |
|---|---|---|---|---|---|---|
| | **Non treated** | | | **Treated** | | |
| | **Mouse number** | **Measure** | **Repopulation %** | **Mouse number** | **Measure** | **Repopulation %** |
| **1 month after implantation** | **166** | 1 | 2.264 | **162** | 1 | 17.035 |
| | | 2 | 2.962 | | 2 | 21.855 |
| | | 3 | 0.312 | | 3 | 11.274 |
| | | mean | 1.846 | | mean | 16.721 |
| | | | | | | |
| | **163** | 1 | 2.373 | **176** | 1 | 17.699 |
| | | 2 | 3.301 | | 2 | 10.178 |
| | | 3 | 8.495 | | 3 | 20.640 |
| | | mean | 4.723 | | mean | 16.172 |
| | | | | | | |
| | **160** | 1 | 1.269 | **161** | 1 | 26.399 |
| | | 2 | 4.924 | | 2 | 14.861 |
| | | 3 | 1.558 | | 3 | 16.021 |
| | | mean | 2.58 | | mean | 20.09 |
| | | | | | | |
| | **mean** | **13.1 ± 1.5** | | **mean** | | **17.7 ± 2.1** |
| | | | | | | |

| | **Non treated** | | | **Treated** | | |
|---|---|---|---|---|---|---|
| | **Mouse number** | **Measure** | **Repopulation %** | **Mouse number** | **Measure** | **Repopulation %** |
| **3 months after implantation** | **153** | 1 | 3.009 | **144** | 1 | 29.186 |
| | | 2 | 10.620 | | 2 | 28.154 |
| | | 3 | 3.349 | | 3 | 29.740 |
| | | mean | 5.659 | | mean | 29.026 |
| | | | | | | |
| | **136** | 1 | 3.393 | **131** | 1 | 26.979 |
| | | 2 | 10.653 | | 2 | 24.793 |
| | | 3 | 0 | | 3 | 14.161 |
| | | mean | 4.682 | | mean | 21.977 |
| | | | | | | |
| | **155** | 1 | 27.495 | **143** | 1 | 32.229 |
| | | 2 | 0 | | 2 | 26.799 |
| | | 3 | 0 | | 3 | 29.920 |
| | | mean | 9.165 | | mean | 29.884 |
| | | | | | | |
| | **mean** | | **6.5 ± 2.4** | **mean** | | **26.9 ± 4.2** |

## Claims

1. A method of making a chimeric murine model comprising:
a. obtaining an immunocompromised murine host, which carries a transgene or a genetic defect responsible for murine hepatocyte death,
b. implanting human hepatocytes therein,
c. controlling non-adaptive defences,
d. recovering a chimeric murine model harbouring settled human hepatocytes capable of maintaining, differentiating and growing.

2. A method according, wherein the steps of implanting human hepatocytes and controlling non-adaptive defences are inverted.

3. A method according to anyone of claims 1 to 2, wherein said transgene or genetic defect, responsible for murine hepatocyte death, is present at least in one copy in said murine host.

4. A method according to anyone of claims 1 to 3, wherein said implanted hepatocytes can be derived from a donor liver, from a partial hepatectomy, from tissues surrounding tumour, from a cell culture or from a tissue matrix.

5. A method according to anyone of claims 1 to 4, wherein human hepatocytes are non-infected, non-tumoral cells.

6. A method according to anyone of claims 1 to 5, wherein human red blood cells are implanted in the murine host, in addition to human hepatocytes.

7. A method according to anyone of claims 1 to 6, wherein the control of non-adaptive defences corresponds to a reduction of macrophages/monocytes and/or NK cells as measured by FACS analysis.

8. A method according to anyone of claims 1 to 7, wherein macrophages are depleted by controlled administration of a toxic compound in the murine host.

9. A method according to claim 8, wherein the administered toxic compound for macrophage depletion is the dichloromethylene diphosphonate (Cl₂MDP).

10. A method according to anyone of claims 8 to 9, wherein the toxic compound is contained in liposomes, which are injected in murine host.

11. A method according to claim 10, wherein Cl₂MDP containing liposomes are injected at 4 day interval, starting two days after implanting.

12. A method according to anyone claims 1 to 11, wherein NK cells are depleted by administering NK antagonists.

13. A method according to anyone of claims 1 to 12, wherein NK cells are depleted by administering anti-NK antibodies.

14. A method according to claim 13, wherein the anti-NK antibodies are injected at monthly interval, starting two days after implanting.

15. A method according to anyone of claims 1 to 14, wherein said transgene or genetic defect responsible for murine hepatocyte death, is the Alb-uPA transgene.

16. A method according to anyone of claims 1 to 15, wherein the murine host is a mouse

17. A method according to claim 16, wherein the immunocompromised mouse host is selected from the group of:
- SCID mouse (severe combined immunodeficiency),
- SCID/Nod mouse (severe combined immunodeficiency/non obese diabetic),
- BXN (NIHIII or Beige Xid Nude) mouse,
- RAG mouse,
- RAG2 mouse,
- RAG-γC mouse.

18. The method according to claim 17, wherein the mouse host is obtained by:
a. crossing homozygous Alb-uPA transgenic mouse with homozygous SCID mouse;
b. making selective backcrosses;
c. recovering a homozygous SCID, hemizygous Alb-uPA mouse host.

19. A chimeric murine model which is an immunocompromised murine host harbouring at least one transgene or genetic defect responsible for murine hepatocyte death, which non-adaptive defences are controlled and which is implanted with human hepatocytes, said control of non-adaptive defences enable said hepatocytes to maintain, differentiate and grow.

20. A chimeric murine model according to claims 19, wherein said settled human hepatocytes secrete specific proteins for at least 3 months.

21. A chimeric murine model according to anyone of claims 19 to 20, wherein said settled human hepatocytes are receptive to hepatotropic pathogens.

22. A chimeric murine model according to claim 21, wherein hepatotropic pathogens are *Plasmodium* strains, HBV or HCV.

23. A chimeric murine model according to anyone of claims 19 to 22, wherein said transgene or genetic defect responsible for murine hepatocyte death, is the Alb-uPA transgene.

24. A chimeric murine model according to anyone of claims 19 to 23, wherein the murine host is a mouse.

25. A chimeric mouse model according to claim 24, wherein the immunocompromised mouse host is selected from the group of:
- SCID mouse (severe combined immunodeficiency),
- SCID/Nod mouse (severe combined immunodeficiency/non obese diabetic),
- BXN (NIHIII or Beige Xid Nude) mouse,
- RAG mouse,
- RAG2 mouse,
- RAG-γC mouse.

26. A chimeric murine model according to claim 25, wherein the mouse host is a homozygous SCID, hemizygous Alb-uPA mouse.

27. A chimeric murine model according to anyone of claims 19 to 26, wherein the murine host is implanted with human red blood cells.

28. A method for studying a hepatotropic pathogen, in a chimeric murine model according to anyone of claims 19 to 27 comprising:
a. infecting said chimeric murine model with a hepatotropic pathogen, in conditions enabling said pathogen to penetrate the settled human cells of the chimeric murine model,
b. observing the pathogen-generated infection in said settled cells.

29. A method according to claim 28, wherein the infection is observed by light microscopy, by immunofluorescence antibody test (IFAT) using pathogen specific antibodies or by RT-PCR using primers for a pathogen specific gene.

30. A method according to anyone of claims 28 to 29, wherein the number of pathogens calculated by light microscopy, the staining obtained by IFAT and the transcripts detected in the settled human cells are compared to those of a control murine animal, infected by the same pathogen but devoid of cell implantation.

31. A method according to claim 30, wherein the hepatotropic pathogen is chosen among *Plasmodium* strains, HBV or HCV.

32. A method according to claim 31, wherein *Plasmodium falciparum* is used for infection and infection is observed by antibodies, specific of *Plasmodium falciparum* liver forms, or by nucleotide sequence amplification using primers specific for *Plasmodium falciparum* liver form genes.

33. A method according to claim 32, wherein said specific antibodies recognize the LSA-1 protein, or wherein said primers enable amplification of the LSA1 gene.

34. Use of a chimeric murine model according to claims 19 to 27 for the testing of a compound for a potential therapeutic interest.

35. A method for screening active compounds against the infection by a hepatotropic pathogen or against its detrimental effects in a chimeric murine model according to anyone of claims 19 to 27 comprising:
a. infecting said chimeric murine model with a hepatotropic pathogen, in conditions enabling said pathogen to penetrate the settled human cells of the chimeric murine model;
b. administering the tested compound in conditions allowing its activity;
c. observing the effects of said compound on the pathogen-generated infection or on its detrimental effects.

36. A method according to claim 35, wherein the infection is observed by light microscopy, by immunofluorescence antibody test (IFAT) using pathogen specific antibodies or by RT-PCR using primers for a pathogen specific gene.

37. A method according to anyone of claims 35 to 36, wherein the number of pathogens calculated by light microscopy, the staining obtained by IFAT and the transcripts detected in the settled cells are compared in the same chimeric murine model at different time points.

38. A method according to claims 37, wherein the hepatotropic pathogen administrated to the chimeric murine model is chosen among *Plasmodium* strains, HBV or HCV.

39. A method according to claim 38, wherein *Plasmodium falciparum* is used for infection and infection is observed by antibodies, specific of *Plasmodium falciparum* liver forms, or by nucleotide sequence amplification using primers specific for *Plasmodium falciparum* liver form genes.

40. A method according to claim 39, wherein said specific antibodies recognize the LSA-1 protein, or wherein said primers enable amplification of the LSA1 gene.

41. A method for screening the *in vivo* metabolism of xenobiotic compounds, in a chimeric murine model according to anyone of claims 19 to 26 comprising:
a. administrating the xenobiotic compound to be tested to said chimeric murine model in conditions allowing the compound to interact with settled human hepatocytes,
b. observing its biotransformation by said settled hepatocytes.

42. A method according to claim 41, wherein the compound biotranformation is evaluated by the detection and/or measurement of the level of metabolites prior and after administration of the compound.

43. A method according to anyone of claims 41 to 42, wherein toxic effects on human hepatocytes are evaluated and where the appropriate compound doses, at which the effects appear, calculated.

44. A method according to anyone of claims 41 to 43, wherein potential interactions between reactive metabolites and cellular macromolecules are studied.

45. A technical platform, useful to identify new compounds useful to treat mammal infections provoked by a hepatotropic pathogen, **characterized in that** it comprises at least a chimeric murine model according to anyone of claims 19 to 27 and appropriate means to detect or to observe the effects of said compounds on a pathogen-generated infection of said murine model.

46. A technical platform, useful for screening the *in vivo* metabolism of xenobiotic compounds **characterized in that** it comprises at least a chimeric murine model according to anyone of claims 19 to 27 and appropriate means to observe the biotransformation of said compounds by implanted human hepatocytes in said murine model.
